# EUROPEAN PATENT APPLICATION

(11) **EP 4 183 283 A1**
(43) Date of publication of application: **24.05.2023**
(21) Application number: 22198249.9
(22) Date of filing: 28.09.2022
(51) Int. Cl.: A41D 27/28, A41D 31/18, A43B 1/00, A43B 1/05, A43B 7/08, B32B 5/24, D06N 7/00, D06P 5/30, A43B 23/02, B29C 65/02, A41C 3/00, A41C 3/14, A41D 1/08, A61F 13/08, B41M 5/00

(54) **TEXTILE FOR SUPPORTING HUMAN MOTOR ORGANS**

(30) Priority: 22.10.2021 TW 110139391
(71) Applicant: Chance Line Industrial Co., Ltd., Siansi Township, Changhua County (TW)
(72) Inventor: YAO, MING-HSIEN, Siansi Township (TW); SHIH, PO-JEN, Siansi Township (TW)
(74) Representative: Lermer, Christoph

(57) **Abstract**

A textile (50) comprising a supporting mechanism (40, 40A, 40B, 40C) disposed on a surface of a fabric (51), having a printed layer (32) and a layered elastic support (42); the elastic support is made of thermoplastic elastomer, and is connected with the printed layer; and a plurality of air-permeable elements (37, 38) disposed in the supporting mechanism. The textile of the invention can be made into wearables for a human body, and the supporting mechanism is capable of supporting human motor organs.

## Description

### BACKGROUND OF THE INVENTION

### Field of Invention

The invention is related to textiles, and specifically refers to a supportive textile capable of providing support and protection for human motor organs.

### Related Art

Garments (such as pressure garments, pressure trousers, and corsets) developed by the current techniques have restraint function to support the muscles and joints of the human body, provide protection for the body, and facilitate fitness exercises, or to shape the human body.

There are three main types of restraint or supportive garments, the first type is made by weaving with elastic fibers. For example, 20% elastic fibers and 80% general fibers are woven into a pressure garment (elastic garments), the elastic fibers are woven with a higher density in the support parts, and are woven with a lower density in the unsupported parts, and the elastic fibers are used to create pressure differences. The restraint textile woven with elastic fibers is not ideal for support of torso, muscles or joints of the human body, after the textile is stretched, or after washing and wearing, the resilience (fatigue resistance) of the elastic fibers gradually decreases, and the size of the textile becomes larger, so the support effect will gradually decrease. In addition, the elastic fibers in the textile are woven in one direction. In the weaving direction of the elastic fibers, the textile has elasticity. In other directions, the textile only has the stretchability formed by the woven structure, so the support of the textile woven with the elastic fibers is not comprehensive, and only a certain direction is elastic. Furthermore, the manufacturing cost of elastic fibers is high and many various manufacturing procedures are required, which is not conducive to energy saving and carbon reduction.

The second type involves sewing pieces of cloth made with elastic fibers onto the fabric. This type of garments have the same disadvantages of elastic fatigue and higher cost.

The third type is to sew or hot-press a sheet or strip of plastic material onto the garment and use the plastic material to provide support. Although the support of this type of garments is better than that of the woven type garments, this type of garments is thick, the pressure is too concentrated, it is not easy to put on and take off, the air permeability of the plastic material is not ideal, and it is hot and airtight and discomfortable to wear. In addition, the manufacturing process of plastic material is complicated, and will generate a lot of waste and cause environmental pollution. The pollution generated includes wastes produced after the plastic material is cut into the desired shape, and pollutants from dyeing.

Dyeing any material such as cloth and leather requires a lot of water, and the industrial wastewater after dyeing and finishing causes great pollution to the environment. The high cost of wastewater treatment equipment increases the manufacturing cost of the product.

Wearables that support the human body also include bras and shoes. Bras support the breasts with built-in cups, wherein the cups are formed by joining multiple pieces of fabric cut into specific shapes. Many various procedures and a lot of manpower are required for manufacturing the cups. Another way of making bra cups is to make a semi-finished product from a piece of fabric using elastic fibers, and hot-press the semi-finished product into a cup shape. The hot-pressing temperature is 185~195°C, and the hot-pressing time is 120 seconds. The temperature of the hot-pressing process is high, the production speed is slow, and it is easy to produce defective products. The high temperature of hot-pressing can damage the fibers and cause fiber cracking. In order to provide pressure and support, all the currently available supportive fabrics are heavy, resulting in a huge waste of materials, as well as manpower and electricity consumption in the manufacturing process.

As far as shoes are concerned, shoes made of knitted fabrics have poor support for the feet due to the large degree of deformation of the circular knitted and flat knitted fabrics. Shoes made of leather are capable of enveloping the feet, but leather shoes are stuffy and airtight, and the manufacturing process of leather shoes is not friendly to the environment. The manufacturing process of leather involves the use of solvents, cutting the leather, joining the cut pieces, and dyeing the leather. The manufacturing process of leather shoes is complicated and the manufacturing speed is slow, and as mentioned above, dyeing harms the global environment, and the solvents of the leather also cause great damage to the environment.

At present, many bra cups or shoes made of textile are reinforced with plastic cut pieces, but the material loss caused by cutting plastic is very large. In addition, if the plastic for reinforcement is combined with the textile by high temperature pressing, the color of the dye on the textile will migrate to the plastic for reinforcement. For example, the color of the black textile will migrate to the plastic to affect the appearance of the textile.

Fabrics used in the vamps of sports shoes are first dyed, then cut, sewed, or bonded. The manufacturing process involves many steps, the manufacturing speed is slow, and dyeing also causes pollution.

Some shoes are patterned by sublimation, patterns formed by sublimation are not clear and detailed, and cannot be made into a three-dimensional (3D) pattern.

The above-mentioned various kinds of garments or wearables supporting the human body require multiple manufacturing processes, which result in high carbon emissions, high manufacturing costs and pollution. Some process wastes cannot be recycled, even if some wastes can be recycled, the recycling process will still cause pollution. For example, for recycled plastic or leather, strong acid must be used to remove the solvent, and the recycling process will still cause pollution.

### SUMMARY OF THE INVENTION

An object of the invention is to provide a textile for supporting human motor organs, so that wearables made of the textile have an innovative supportive structure to support and protect the motor organs of human body.

Another object of the invention is to provide a textile for supporting human motor organs, and a method applied for manufacturing a structure of the textile is environmentally friendly and capable of reducing pollution.

Yet another object of the invention is to provide a textile for supporting human motor organs, which has fewer manufacturing procedures, and is capable of saving energy, reducing carbon emissions, and reducing manpower.

Yet another object of the invention is to provide a textile for supporting human motor organs, and a supporting mechanism of the textile has a color and does not need to be dyed.

An object of the invention is to provide a supportive thermoprinting material, the thermoprinting material is combined with the textile in order to make the textile for supporting human motor organs according to the above-mentioned various objects.

The invention provides a textile for supporting human motor organs, comprising:
a fabric;
at least one supporting mechanism combined onto at least one surface of the fabric, having a printed layer and a layered elastic support;
the printed layer being formed by resin printing;
the elastic support being made of thermoplastic elastomer, its shape being consistent with the printed layer, and being combined with the printed layer; the printed layer and the elastic support being combined/bonded on the fabric together; and
a plurality of air-permeable elements located within a disposing range of the supporting mechanism.

The textile of the invention can be made into wearables for the human body, the supporting mechanism has elastic pressure and tensile elasticity, is capable of supporting and protecting torso, muscles, joints, core muscle groups, bones and other motor organs of the human body, helping muscle groups to restore and helping people to be more labor-saving and convenient in sports and fitness, and reducing the occurrence of sports injuries. The air-permeable elements of the supporting mechanism make the textile air-permeable for moisture circulation.

In the structure of the textile of the invention, the supporting mechanism is formed by the printed layer and the layered elastic support, and a manufacturing process is more environmentally friendly, manufacturing procedures can be reduced, does not generate pollutants, and has an effect of energy saving and carbon reduction. The color of the supporting mechanism can be obtained without the conventional dyeing process, without wasting water resources and without the need for waste water treatment equipment.

In one embodiment, the at least one supporting mechanism is disposed on a surface of the fabric. In one embodiment of the invention, at least two supporting mechanisms are provided and are respectively disposed on two surfaces of an inner side and an outer side of the fabric. The supporting mechanism on the surface of the inner side of the fabric further provides an anti-slip effect to fix the muscles.

The supporting mechanism is formed by non-line-shaped supports or line-shaped supports, and the non-line-shaped support can be a monolithic support or an area-shaped support.

A plurality of adjacent monolithic supports are capable of forming the supporting mechanism, and there is an air-permeable gap between the two adjacent monolithic supports.

One area-shaped support or a plurality of area-shaped supports is/are capable of forming the supporting mechanism, each of the area-shaped supports has a plurality of air-permeable holes; there are air-permeable gaps between the adjacent area-shaped supports. Lines in at least two directions can be connected to form a plurality of grids, thereby enhancing a structural strength of the supporting mechanism.

The textile is a fabric or a wearable made for the human body, such as clothes, trousers, shoes, and protective gear such as knee pads, elbow pads or wrist pads, which provide support and protection for the human motor organs.

The printed layer can be colored or colorless. The thermoplastic elastomer is: polyurethane thermoplastic elastomer (TPU), polyamide thermoplastic elastomer (TPAE), polyester thermoplastic elastomer (TPEE) or polyolefin thermoplastic elastomer (TPO).

The invention further provides a supportive thermoprinting material comprising:
a substrate, one surface thereof being a release surface;
a printed layer printed on the release surface of the substrate; a plurality of air-permeable elements being formed within a printing range of the printed layer; and
a thermoplastic elastomer material layer capable of generating adhesion when being heated, the thermoplastic elastomer material layer being disposed on the printed layer and without covering the air-permeable elements.

The printed layer and the thermoplastic elastomer material layer of the thermoprinting material are hot-pressed on a fabric to make the textile of the invention.

The thermoplastic elastomer material layer is combined with the printed layer in a powder or granular form. In one example of the invention, the thermoplastic elastomer material layer is combined with the printed layer before the printed layer becomes dry.

The printed layer is a plurality of monolithic printed bodies, or at least one area-shaped printed body, or at least one line-shaped printed body. The air-permeable elements are air-permeable gaps or air-permeable holes.

### BRIEF DESCRIPTION OF THE DRAWINGS

The objects, features, and achieved efficacies of the invention can be understood from the description and drawings of the following preferred embodiments, in which:
FIGS. 1A and FIG. 1B are schematic diagrams of an outer surface and an inner surface respectively of a textile of a preferred embodiment of the invention.
FIG. 2 is a perspective view of an example of a supportive thermoprinting material of a first preferred embodiment of the invention.
FIG. 3 is a cross-sectional view of FIG. 1.
FIG. 4 is a perspective view of another example of the supportive thermoprinting material of the first preferred embodiment of the invention.
FIG. 5 is a cross-sectional view taken along section line 5-5 of FIG. 4.
FIG. 6 is a cross-sectional view showing the thermoprinting material of FIG. 2 adhered on a fabric.
FIG. 7 is a cross-sectional view of an example of the textile with a supporting mechanism of the first preferred embodiment of the invention.
FIG. 8 is a cross-sectional view showing the thermoprinting material of FIG. 4 adhered on a fabric.
FIG. 9 is a cross-sectional view of another example of the textile with the supporting mechanism of the first preferred embodiment of the invention.
FIG. 10 is a photograph of a real object of the textile with the supporting mechanism of the invention.
FIG. 11 is a partial enlarged view of FIG. 10.
FIGS. 12A and 12B are photographs of a real object of the textile of FIG. 10 and showing the textile being stretched.
FIG. 13 is a perspective view of an example of the supportive thermoprinting material of a second preferred embodiment of the invention.
FIG. 14 is a cross-sectional view taken along section line 14-14 of FIG. 13.
FIG. 15 is an example of a line-shaped printed body of the thermoprinting material of FIG. 13.
FIGS. 16A to 16H show different examples of the line-shaped printed body.
FIGS. 17A to 17C are schematic diagrams of the textile with the supporting mechanism of the invention.
FIGS. 18A to 18D are schematic diagrams showing the textile with the supporting mechanism of the invention applied to fitness trousers.
FIG. 19 is a schematic diagram of the textile of the invention applied to sportswear.
FIG. 20 and FIG. 21 are schematic diagrams showing the textile of the invention applied to bra cups.
FIG. 22 is a schematic diagram of the textile of the invention applied to a vamp of a shoe.
FIGS. 23A and 23B are schematic diagrams of the textile of the invention applied to clothes.

### DETAILED DESCRIPTION OF THE INVENTION

The invention provides a textile 50 for supporting and protecting human motor organs, the motor organs refer to muscles, joints, ligaments, tendons and bones related to human movement. The textile 50 can be made into a variety of garments for the human body, including but not limited to: clothes, trousers, socks, silk stockings, gloves, bra cups, knee pads, elbow pads, wrist pads, and various types of shoes and other wearables. The wearables, according to their wearing positions and types, are capable of providing support for torso, muscles, elbows, wrists, knees, ankles and other joints, chest (breasts), feet and bones of the human body, and capable of promoting implementation of fitness and sports. The wearables made of the textile 50 restrict a range of motion of muscles by means of anti-slip and/or pressure to avoid or reduce a chance of injury to athletes. The wearables can be used to shape an upper body or a lower body of a person, and can also be used to control a flow direction of human blood.

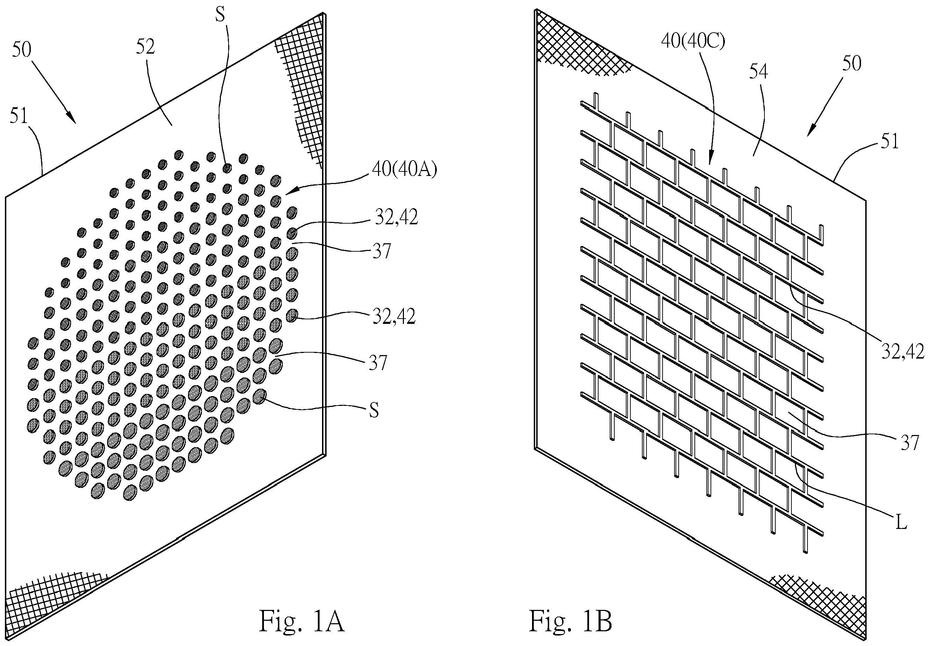

Please refer to FIG. 1A and FIG. 1B, the invention can provide a supporting mechanism 40 (40A, 40C) on an outer surface 52 and an inner surface 54 of a fabric (e.g., cloth 51) woven with various artificial or natural fibers or yarns to form the textile 50 with the supporting mechanism 40 of the invention. The inner surface 54 refers to a surface of the fabric (cloth 51) that contacts a human body, and the outer surface 52 refers to an exposed surface of the cloth 51 facing outwardly. The supporting mechanism 40A of FIG. 1A is a non-line-shaped configuration, while the supporting mechanism 40C of FIG. 1B is a line-shaped configuration. The supporting mechanism 40 referred to in this specification comprises the supporting mechanisms 40A, 40B in non-line-shaped form and the supporting mechanism 40C in line-shaped form.

The invention further provides a thermoprinting material 10 (10A, 10C), and a preset mechanism 30 (30A, 30B, 30C) of the thermoprinting material 10 is combined with a fabric (such as garments, trousers or cloth) by hot-pressing to make the textile 50 with the supporting mechanism 40 of the invention.

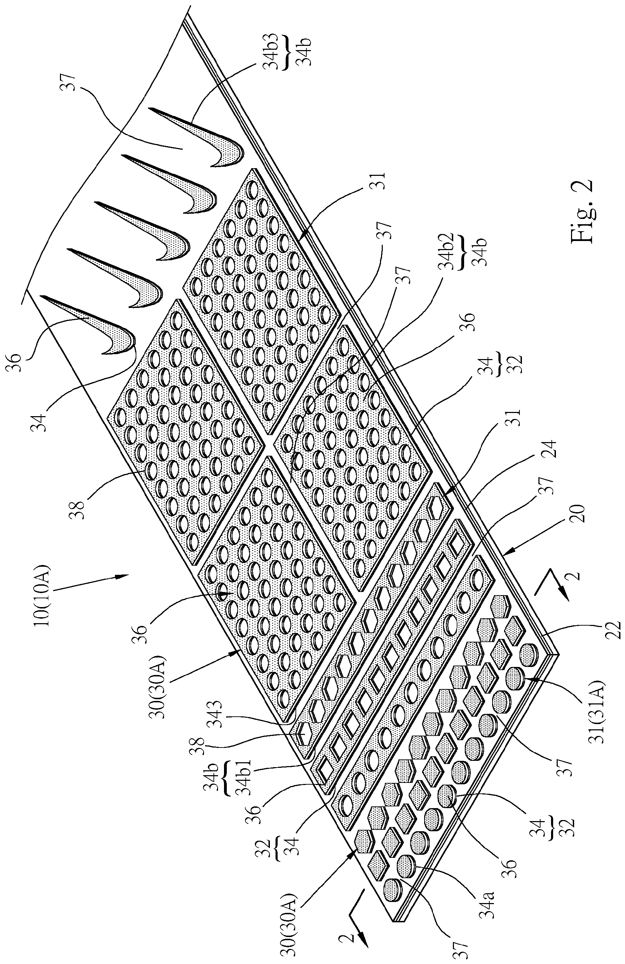

FIGS. 2 to 5 are schematic diagrams of the supportive thermoprinting material 10 (10A) provided by a first preferred embodiment of the invention, which comprises: a substrate 20, one surface of the substrate 20 is a release surface; and the preset mechanism 30 (30A) provided on the release surface of the substrate 20.

The substrate 20 is a plastic sheet with appropriate rigidity and low extensibility, and capable of withstanding temperatures above 130° C without melting. In this embodiment, a PET (polyethylene terephthalate) film is selected as the substrate 20. A surface of the substrate 20 can be subjected to release treatment to become a release surface. In this embodiment, a release layer 22 is disposed on a surface of the substrate 20 to form the release surface.

The preset mechanism 30 (30A) comprises a resin printed layer (hereinafter referred to as a printed layer) 32 and a thermoplastic elastomer material layer 36, and the printed layer 32 is digitally printed on the release surface of the substrate 20, that is, printed on the release layer 22. In order to increase a bonding between the printed layer 32 and the release surface, in this embodiment, a bonding promotion layer 24 is further coated on the release layer 22, and an adhesion of the printed layer 32 on the release surface is improved through the bonding promotion layer 24. The bonding promotion layer 24 is made of acrylic resin in this embodiment, but other materials or substances that are capable of promoting a bonding between the printed layer 32 and the release layer can be applied to the invention.

The resin printed layer 32 can be colored ink or non-colored ink, and the ink contains resin components. The ink of the invention can be selected from water-based ink or oil-based ink. The printed layer 32 of this preferred embodiment uses water-based ink, which is suitable for fabrics and clothing, and does not fade when washed with water, and is environmentally safe and harmless to the human body. The water-based ink used in the printed layer 32 contains water, water-based PU (polyurethane) and pigments, and the pigments can be colored or colorless. Depending on the pigments used, the colorless printed layer 32 or the printed layer 32 with various colors can be printed. The printed layer 32 can also be made of a thermoplastic high molecular elastic polymer containing polyurethane and without pigments. In the invention, oil-based ink can also be used, which also contains PU and pigments. By digital printing, the printed layer 32 of various areas, various sizes, various colors and with various patterns or shapes can be accurately printed.

The thermoplastic elastomer material layer (hereinafter referred to as the elastomer material layer) 36 is disposed on the printed layer 32, a first surface of the printed layer 32 is directly or indirectly connected to the release surface of the substrate 20, and a second surface of the printed layer 32 is combined with the elastomer material layer 36. The elastomer material layer 36 is made of thermoplastic elastomer (TPE) with excellent recovery property. The invention uses environmentally friendly thermoplastic elastomer, including but not limited to: polyurethane thermoplastic elastomer (TPU or TPE-U), polyamide thermoplastic elastomer (TPAE), thermoplastic ester elastomer (TPEE), or thermoplastic olefin elastomer (TEO, TPO or TPE-O).

In this preferred embodiment, powdered or fine-grained TPU hot-melt adhesive is used as the thermoplastic elastomer material layer 36, and the TPU hot-melt adhesive powder is coated on the printed layer (e.g., water-based resin) 32 when the printed layer 32 is not dry, still damp and has moisture, the hot-melt adhesive powder is combined with the printed layer 32, and there is no hot-melt adhesive powder where the printed layer 32 is absent. The printed layer 32 and the hot-melt adhesive on the printed layer 32 are dried to shape the printed layer 32 and the hot-melt adhesive to manufacture the thermoprinting material 10. The shaped hot-melt adhesive forms the elastomeric material layer 36. The preset mechanism 30 (30A) is densely distributed with a large number of air-permeable elements after being manufactured, and the air-permeable elements are air-permeable holes or air-permeable gaps.

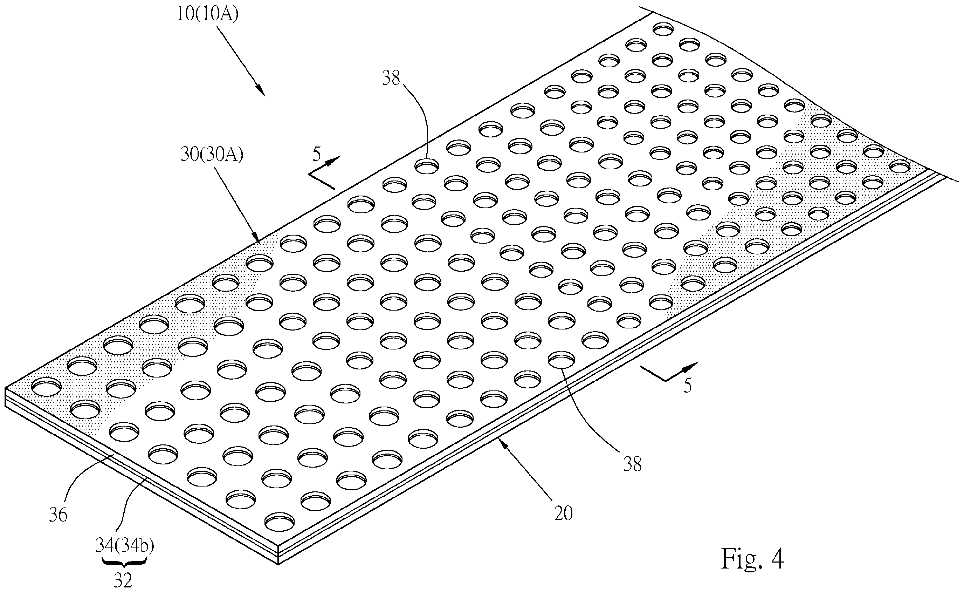

Pease refer to FIG. 2 and FIG. 4, specifically, the printed layer 32 is formed by one non-line-shaped printed body 34 or a plurality of non-line-shaped printed bodies 34, and the non-line-shaped printed body 34 is divided into two forms, one form is an independent monolithic printed body 34a, another form is an area-shaped printed body 34b having a length and a width distinctly, such as a strip-shaped printed body 34b1, a rectangular printed body 34b2, or a printed body 34b3 of a specific pattern as shown in FIG. 2. The invention digitally prints the printed layer 32, and is capable of accurately printing the various non-line-shaped printed bodies 34 constituting the printed layer 32, the printed bodies 34 can be printed in different shapes and sizes as required, and can be printed in geometric, non-geometric, or arbitrarily designed shapes and patterns. Taking FIG. 2 as an example, the monolithic printed body 34a can be a geometric shaped (such as a circle, an ellipse, a rectangle, a polygon) or a non-geometric dot-shaped object; the area-shaped printed body 34b can be various regularly shaped or irregularly shaped printed objects, wherein the printed body 34b3 of a specific pattern can be various known graphics or patterns, such as NIKE's logo. Size and shape of the printed body 34 can be changed according to printing parameters. An air-permeable gap 37 is formed between the two adjacent monolithic printed bodies 34a, and the air-permeable gap 37 is also formed between the two adjacent printed bodies 34b3. A plurality of air-permeable holes 38 are densely distributed on the area-shaped printed body 34b, and the air-permeable holes 38 can be in geometrical shapes or irregular shapes. A digital printing machine (not shown in the figures) prints the monolithic printed bodies 34a on the release surface of the substrate 20. After printing, the air-permeable gaps 37 are formed between the printed bodies 34a. For the area-shaped printed body 34b, the air-permeable holes 38 are formed at places where the water-based ink is not printed. Shapes or configurations of the non-line-shaped printed body 34 and the air-permeable elements shown in the preferred embodiment of the invention are merely examples rather than limitations. The air-permeable elements are located within a printing area of the printed layer 32.

After printing of the printed layer 32 is completed, the TPU hot-melt adhesive powder is coated on the printed layer 32. The hot-melt adhesive powder is only combined with the printed body 34 containing moisture, and will not adhere to the air-permeable elements (the air-permeable gaps 37 or the air-permeable holes 38), and the air-permeable elements maintain hollow. Thereby, the manufactured preset mechanism 30 naturally forms the air-permeable elements for circulation of air and discharge of sweat or moisture.

Preferably, a width of each of the air-permeable gaps 37 between the two adjacent monolithic printed bodies 34a is not greater than 1mm, for example, more preferably, a width of each of the air-permeable gaps 37 is within a range of 0.2 mm to 0.7mm. Diameter or size of each of the air-permeable holes 38 is preferably not greater than 1 mm, for example, within a range of 0.1 mm to 1 mm, more preferably within a range of 0.2 mm to 0.7 mm. The air-permeable holes 38 at different positions can have different sizes, so as to take into account air permeability and support. Each of the printed bodies 34 and the hot-melt adhesive bonded thereon form a supporting unit 31 (31A). Shape and size of the supporting unit 31A are the same as those of the printed body 34, in the form of dot, bar or area shape. Preferably, a diameter or a width of the dot-shaped supporting unit 31A is between 0.5 mm and 1.5 cm.

Shape, size, and color of the printed layer 32 can be set or changed as required. A density of the printed bodies 34 in the printed layer 32 and a density of the air-permeable elements can also be different. FIG. 1A shows that size and density of a support S in the supporting mechanism 40A are different from those of the air-permeable gap 37.

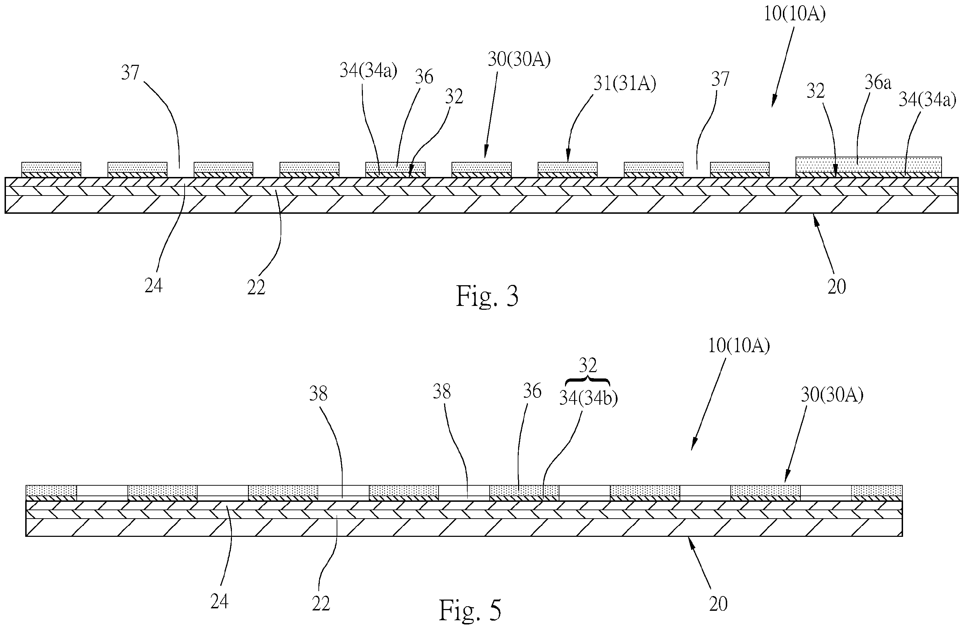

The preset mechanism 30 (30A) can be made with different thicknesses, hardnesses and stiffnesses as required. FIG. 3 shows that an elastomeric material layer 36a on the right side is made with larger size and thickness. By changing composition, thickness, size or purity of the hot-melt adhesive, the preset mechanism 30 with different hardnesses or stiffnesses can be made. Stiffness refers to whether the preset mechanism 30 can be easily stretched and deformed. The greater a stiffness of the preset mechanism 30, the less likely it is to be stretched and deformed.

The hot-melt adhesive powder can be mixed with 0.5-6% graphene or collagen powder, so that the elastomer material layer 36 has compositions of graphene or collagen to provide benefits to the human body.

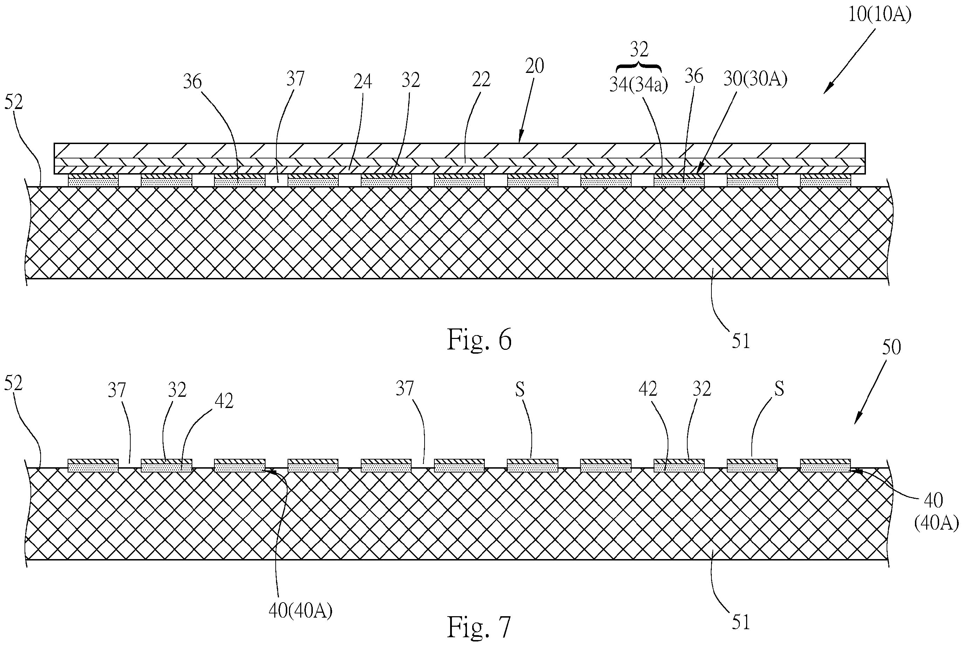

Please refer to FIG. 6, when making the textile 50 with the supporting mechanism of the invention, the thermoprinting material 10 (10A) of FIG. 2 is placed on a fabric, for example, placed on a surface of a cloth 51 or a garment, the elastomeric material layer 36 contacts the outer surface 52 of the cloth 51, and the thermoprinting material 10 (10A) is heated with an iron or a blancher, so that the elastomer material layer 36 of the hot-melt adhesive material melts and produces adhesion and penetrates into fibers of the cloth 51 to combine with the cloth 51, the melted hot-melt adhesive is combined with the printed layer 32, and the elastomeric material layer 36 will not affect a color of the printed layer 32. Afterwards, the substrate 20, the release layer 22 and the bonding promotion layer 24 are separated from the preset mechanism 30 (30A) to manufacture the textile 50, as shown in FIG. 7. After separation, the preset mechanism 30 of the thermoprinting material 10 (10A) is thermally bonded with the cloth 51, the thermoplastic elastomer material layer 36 is cooled and shaped and then transformed into a layered elastic support 42, which is distributed on surfaces and in fibers of the cloth 51. The printed layer 32 is combined with the elastic support 42 to form the supporting mechanism 40 (40A). Polyurethane and pigments of the printed layer 32 are mixed into the elastic support 42, so that the elastic support 42 has colors, and the outer surface 52 of the textile 50 forms colors and patterns. If the printed layer 32 is colorless, the elastic support 42 has a color of the material itself. The air-permeable gaps 37 enable air and moisture to circulate, so that air can flow through the air-permeable gaps 37 and gaps in the fibers of the cloth 51.

This example provides the non-line-shaped supporting mechanism 40A, which has a plurality of adjacent non-line-shaped monolithic supports S. As shown in FIG. 1A and FIG. 7, each of the monolithic supports S is composed of the supporting unit 31A, so it contains components of the printed layer 32 and the thermoplastic elastomer; the air-permeable gaps 37 exist between the monolithic supports S, and the air-permeable gaps 37 are densely disposed within a disposing range of the supporting mechanism 40A. The supports S are distributed within a certain range of the outer surface 52, and a shape of each of the monolithic supports S can be a regular or an irregular dot shape. The supporting mechanism 40A of the present example can be fabricated on the outer surface 52 or the inner surface 54 of the textile 50, and is preferably fabricated on the outer surface 52.

The substrate 20 made of PET can be recycled, and there are no dyes or other polluting substances on the substrate 20, so recycling operation will not cause pollution.

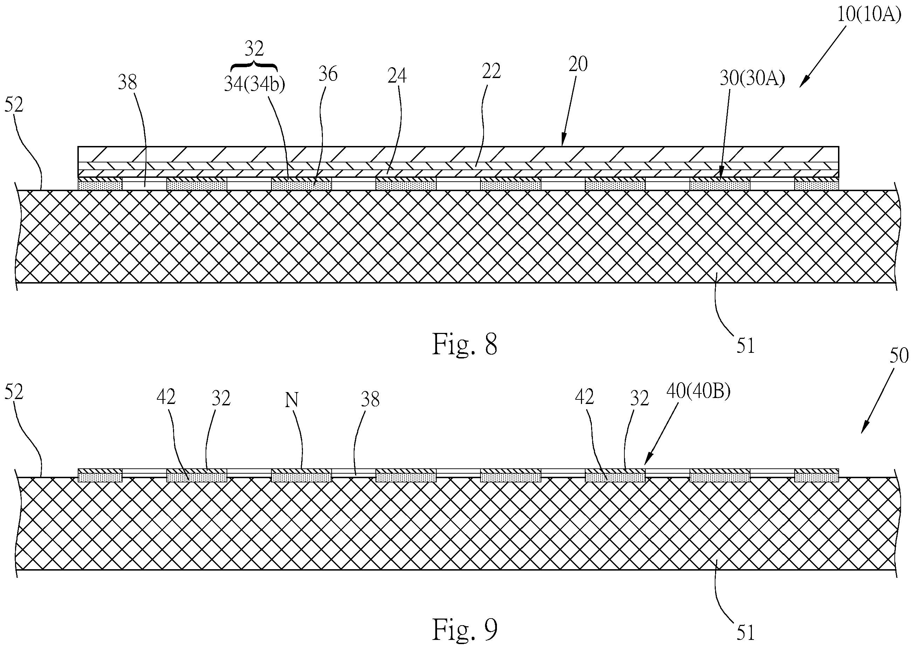

Similarly, referring to FIG. 8, the thermoprinting material 10 (10A) of FIG. 4 is placed on a fabric, for example, placed on an outer surface 52 of a cloth 51, and the thermoprinting material 10 (10A) is heated with an iron or a blancher, so that the elastomer material layer 36 melts and penetrates into the fibers of the cloth 51 to combine with the cloth 51. Afterwards, the substrate 20, the release layer 22 and the bonding promotion layer 24 are separated from the preset mechanism 30 (30A) to form the textile 50 of the invention, as shown in FIG. 9. The thermoplastic elastomer material layer 36 is cooled and shaped and then transformed into the layered elastic support 42, which is distributed on the surfaces and in the fibers of the cloth 51. The printed layer 32 is combined with the elastic support 42 to form the supporting mechanism 40 (40B), and the air-permeable holes 38 are densely disposed within a disposing range of the supporting mechanism 40B for circulation of air and moisture.

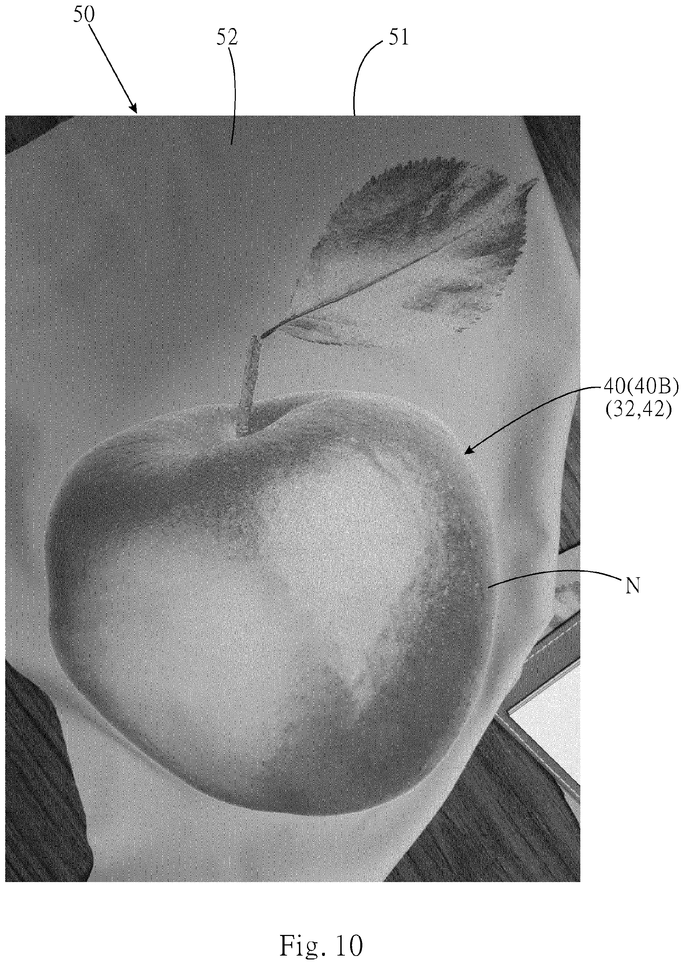

This example provides the area-shaped supporting mechanism 40B, which is formed by one or more than one non-line-shaped and area-shaped supports N, each of the area-shaped supports N is composed of the area-shaped printed body 34b and the elastomeric material layer 36 on the printed body 34b, and the air-permeable holes 38 formed on the supporting mechanism 40B can be in regular or irregular shape. FIGS. 10, 20, 21 and 22 show the supporting mechanism 40B in the form of the area-shaped supports N, each of the supports N has a certain area on the outer surface 52 to provide support and protection for the motor organs of human body.

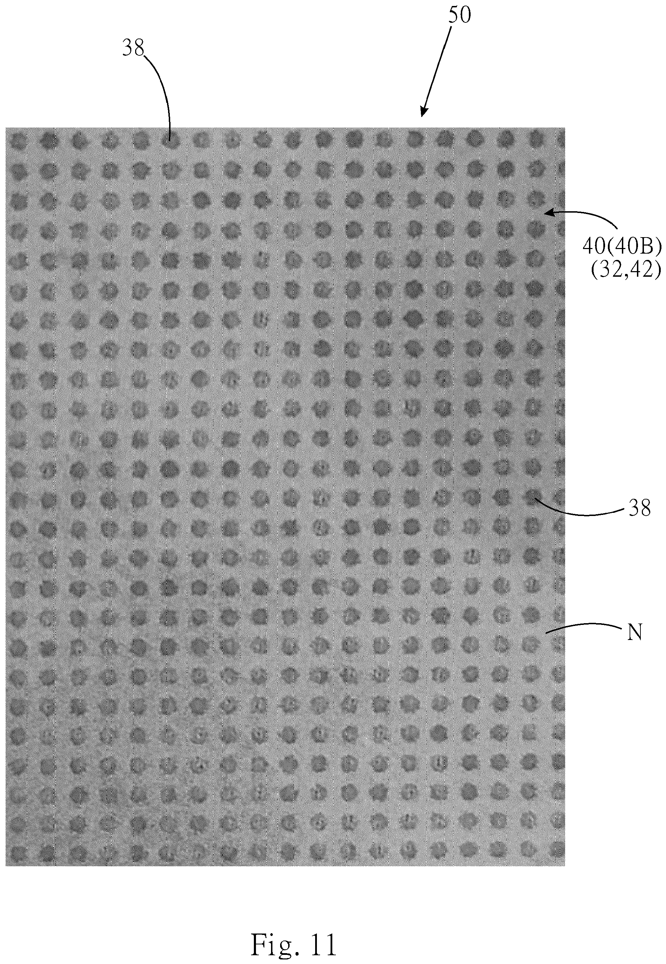

FIG. 10 is a photograph of a real object of the textile 50 with the supporting mechanism of the invention, showing that the supporting mechanism 40B is fabricated on the textile 50 and formed into a pattern. Wherein the printed layer 32 is printed with an apple pattern, and shape, contour and size of the elastic support 42 are the same as those of the printed layer 32. As shown in FIG. 11, the elastic support 42 and the printed layer 32 constitute the resilient supporting mechanism 40B of the textile 50, and the air-permeable holes (air-permeable elements) 38 are densely distributed in the supporting mechanism 40B and penetrate through the elastic support 42 and the printed layer 32 to become channels for air to circulate and to discharge moisture and sweat. The textile 50 with the supporting mechanism 40B of the invention has excellent air permeability and is not stuffy.

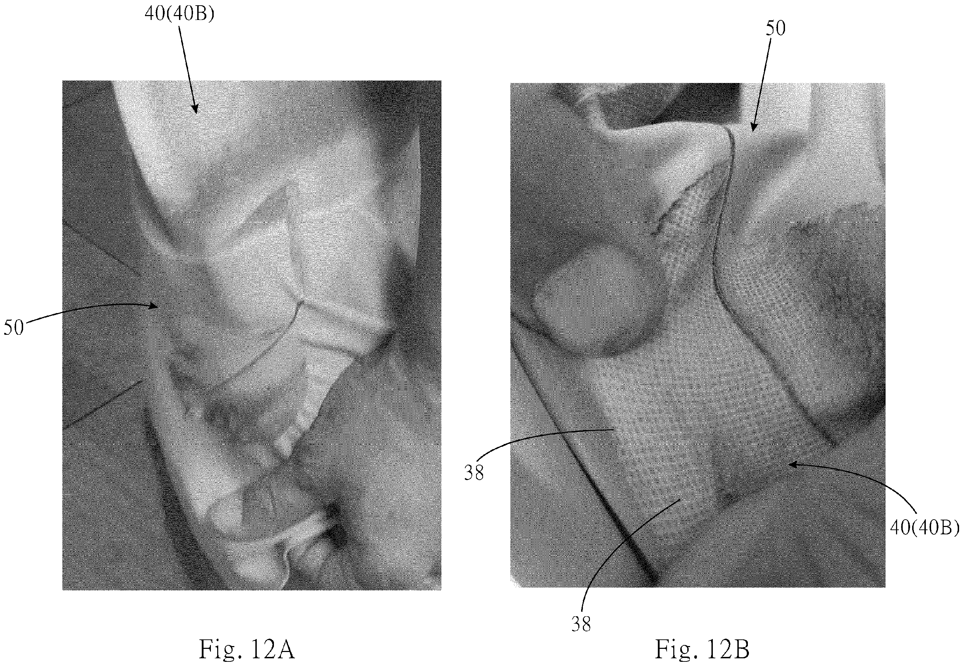

FIGS. 12A and 12B show a state of the supporting mechanism 40 (40B) of the textile 50 being stretched. The elastic support 42 is a thermoplastic elastomer with excellent recovery property, and has excellent recovery property after being stretched. When the textile 50 is made into clothes, trousers or protective gear, wrapping effect and restraining effect of the elastic support 40 are capable of providing excellent support for muscles or joints. According to the inventor's test, the elastic support 42 has a recovery rate of 99% after being stretched, and is capable of providing excellent elastic pressure and support. After more than 3,000 tensile tests, the elastic support 42 can still recover and has excellent fatigue resistance. The printed layer 32 of this embodiment is made of pure TPU hot-melt adhesive with high molecular weight, and the elastic support 42 produced has excellent physical properties such as elasticity and recovery.

The printed layer 32 is combined with the elastic support 42 and located on a surface of the textile 50. The printed layer 32 can be printed with various different colors. As shown in FIG. 10, a pattern of the textile 50 has gradient colors.

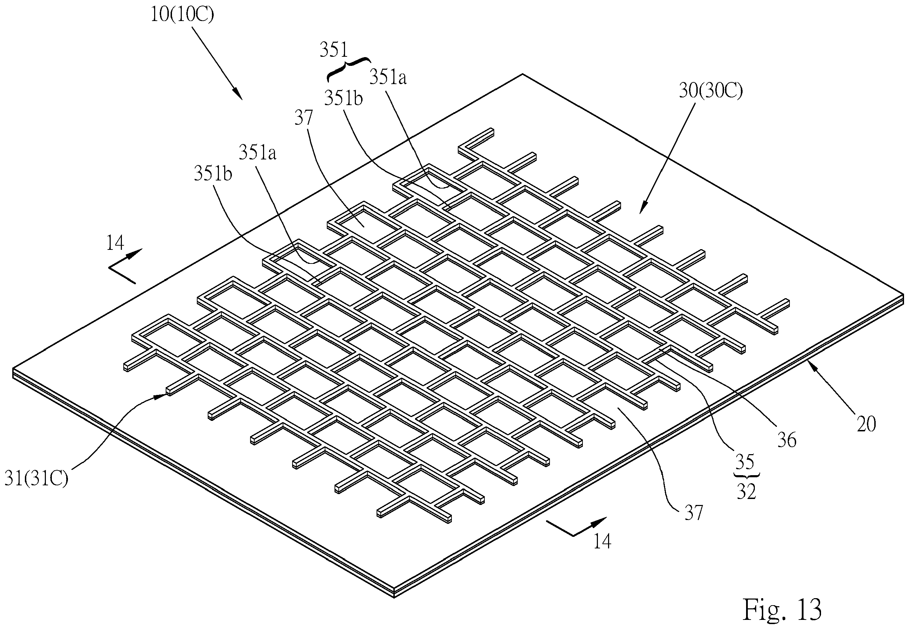

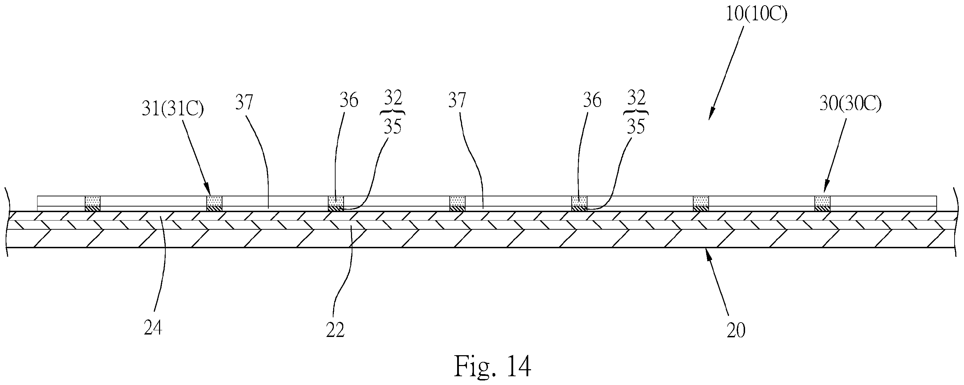

FIGS. 13 and 14 are schematic views of the supportive thermoprinting material 10 (10C) provided by a second preferred embodiment of the invention, which also comprises a substrate 20 and a preset mechanism 30 (30C) disposed on the release surface of the substrate 20. A surface of the substrate 20 can be provided with a release layer 22 as a release surface, and a bonding promotion layer 24 is coated on the release layer 22. The substrate 20, the release layer 22 and the bonding promotion layer 24 can be understood from the first preferred embodiment.

The preset mechanism 30 (30C) comprises the resin printed layer (hereinafter referred to as the printed layer) 32 and the thermoplastic elastomer material layer 36, and the printed layer 32 is digitally printed on the release surface of the substrate 20.

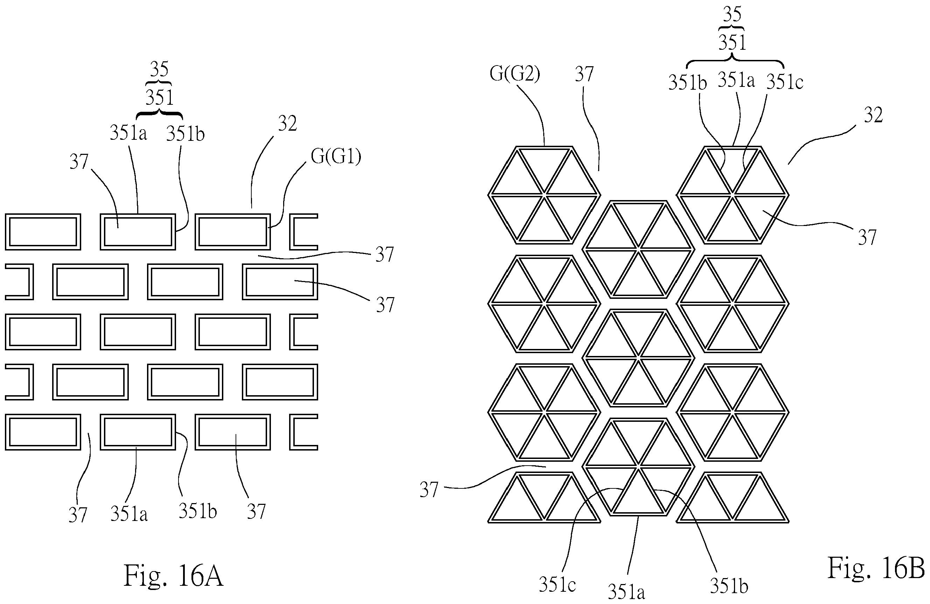

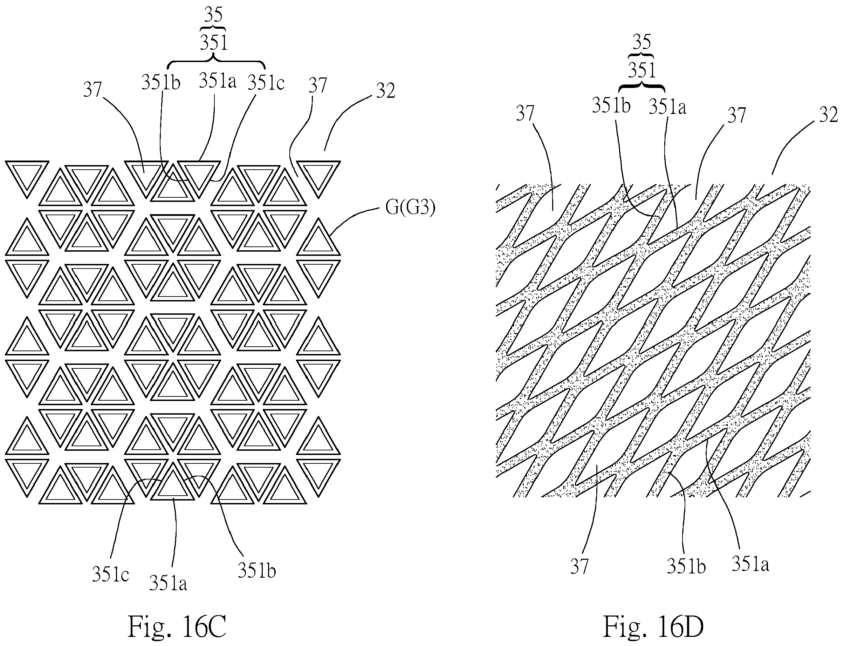

The printed layer 32 is formed by at least one line-shaped printed body 35. Line-shape refers to that a configuration of the printed body 35 uses lines as constituent elements. The printed body 35 has lines 351 densely formed in at least two directions, and the air-permeable gaps 37 are formed between the lines 351. As shown in FIGS. 13 and 16A to 16E, the line-shaped printed body 35 is characterized in an air-permeable area of the air-permeable gaps 37 being larger than an area of the printed lines 351, so an air permeability of the line-shaped printed body 35 is excellent.

The printed body 35 shown in FIG. 13 has lines 351a in a first direction and lines 351b in a second direction, which are disposed in directions perpendicular to each other. The lines 351a, 351b in the two directions are connected with one another. A width of each of the lines 351 ranges from 0.5 mm to 2.5 mm, preferably 1 mm or 2 mm, but is not limited thereto.

After printing of the printed layer 32 is completed, the TPU hot-melt adhesive powder is coated on the printed layer 32. The hot-melt adhesive powder is combined with the printed body 35 through the moisture of the printed body 35, and will not adhere to the air-permeable gaps 37.

The printed body 35 and the hot-melt adhesive bonded thereon form the supporting unit 31 (31C). Shape and size of the supporting unit 31C are the same as those of the printed body 35.

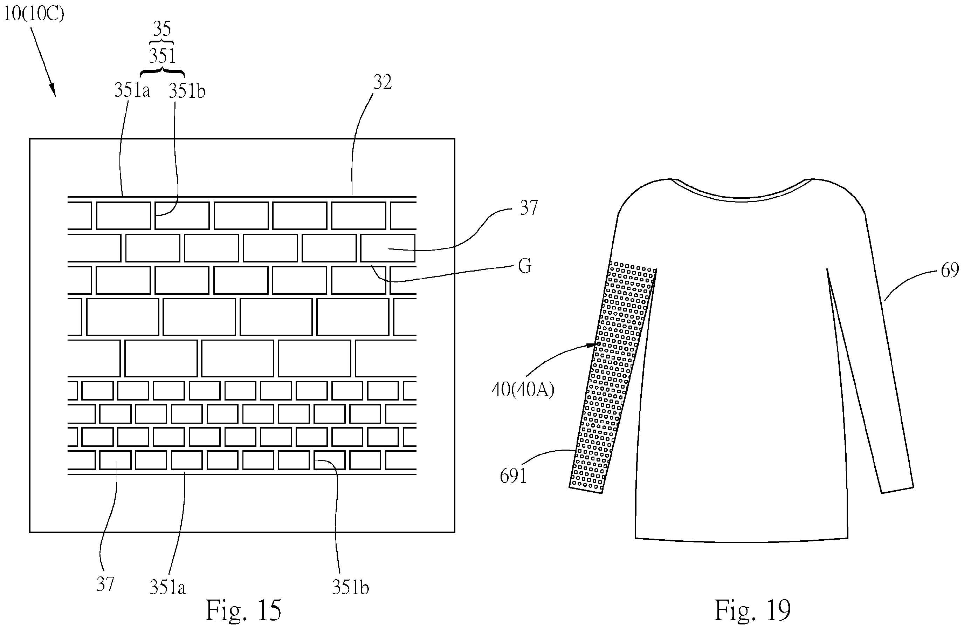

Shape, size, and color of the printed layer 32 can be set or changed as required. A density of the lines 351 of the printed body 35 can be changed as required. As shown in FIG. 15, the lines 351 (351a and 351b) can be printed in different densities at different positions of the printed body 35. At positions with a higher density of the lines 351, the greater the pressure and elasticity formed by the supporting mechanism 40 on the textile 50; on the contrary, at positions with a lower density of the lines 351, the smaller the pressure and elasticity formed by the supporting mechanism 40 on the textile 50.

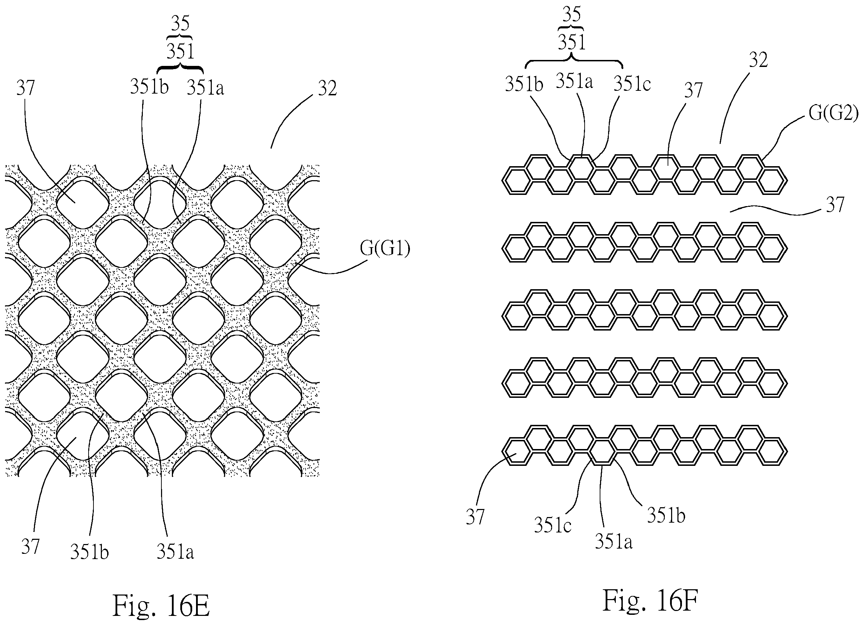

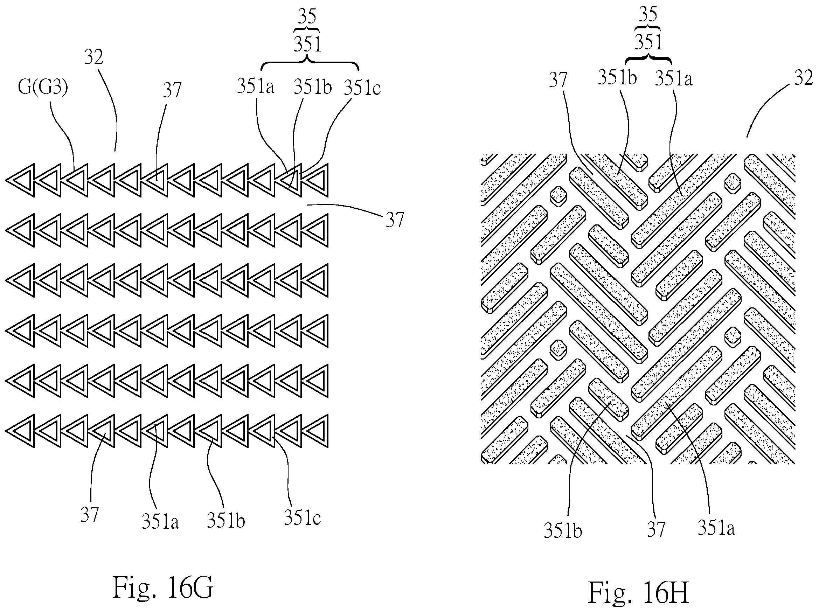

In the printed body 35 shown in FIGS. 13 and 16A to 16G, the lines 351 in different directions are connected with one another to form grids G, so that the supporting mechanism 40 made of the printed body 35 has excellent supporting strength and elastic pressure and elasticity. For example, the lines 351a, 351b of FIGS. 15, 16A and 16E are connected to form brick-shaped, rectangular grids G (G1), while the lines 351a to 351c of FIGS. 16B and 16F are connected to form hexagonal grids G (G2), and the hexagonal grids G (G2) of FIG. 16F are connected in a honeycomb shape, and the lines 351a to 351c of FIG. 16C and FIG. 16G are connected to form triangular grids G (G3). Taking FIG. 16B as an example, each of the hexagonal grids G2 has excellent structural strength through the lines 351a to 351c in different directions in and around the hexagonal grids G2. The grids G1 to G3 are capable of bearing acting force and component force in various directions such as vertical, horizontal and oblique directions, and supporting and protecting the motor organs in all directions. The lines 351a, 351b in different directions in FIG. 16H are also capable of bearing acting force in different directions.

After printing of the printed layer 32 is completed, the TPU hot-melt adhesive powder is coated on the printed layer 32. The hot-melt adhesive powder is only combined with the printed body 35 containing moisture, and will not adhere to the air-permeable elements (the air-permeable gaps 37), and the air-permeable elements maintain open. Thereby, the manufactured preset mechanism 30 (30C) naturally forms air-permeable parts for circulation of air and discharge of sweat or moisture.

The preset mechanism 30 (30C) of the thermoprinting material 10 (10C) of this embodiment is combined with a surface of a cloth 51, such as the inner surface 54, to manufacture the textile 50 shown in FIG. 1B, the surface of the textile 50 has the at least one line-shaped supporting mechanism 40 (40C) formed by densely distributed lines. A manner in which the preset mechanism 30 (30C) being combined with the cloth 51 is the same as that in the previous embodiment. The thermoplastic elastomer material layer 36 of the preset mechanism 30 (30C) is cooled and shaped and then transformed into the layered elastic support 42, which is distributed on the surfaces and in the fibers of the cloth 51. The printed layer 32 is combined with the elastic support 42 to form the supporting mechanism 40 (40C), and the air-permeable gaps 37 are densely disposed within a disposing range of the supporting mechanism 40C for circulation of air and discharge of moisture.

The present embodiment provides the line-shaped supporting mechanism 40C, which has line-shaped supports L, as shown in FIG. 1B, the support L is formed by the supporting unit 31C, so the support L has shape and size the same as those of the supporting unit 31C, and comprises compositions of the printed layer 32 and the thermoplastic elastomer 36; the air-permeable gaps 37 exist between lines of the supports L, and the supports L have a certain area on the inner surface 54 to provide support and protection for human motor organs. Arrangement of line of the line-shaped supports L can be any one shown in FIG. 13, FIG. 16A to FIG. 16H, or other arrangement forms.

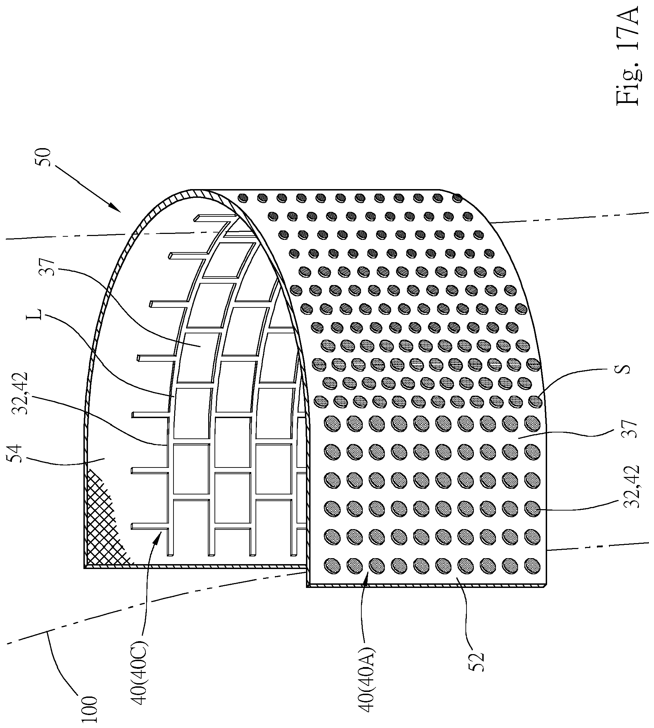

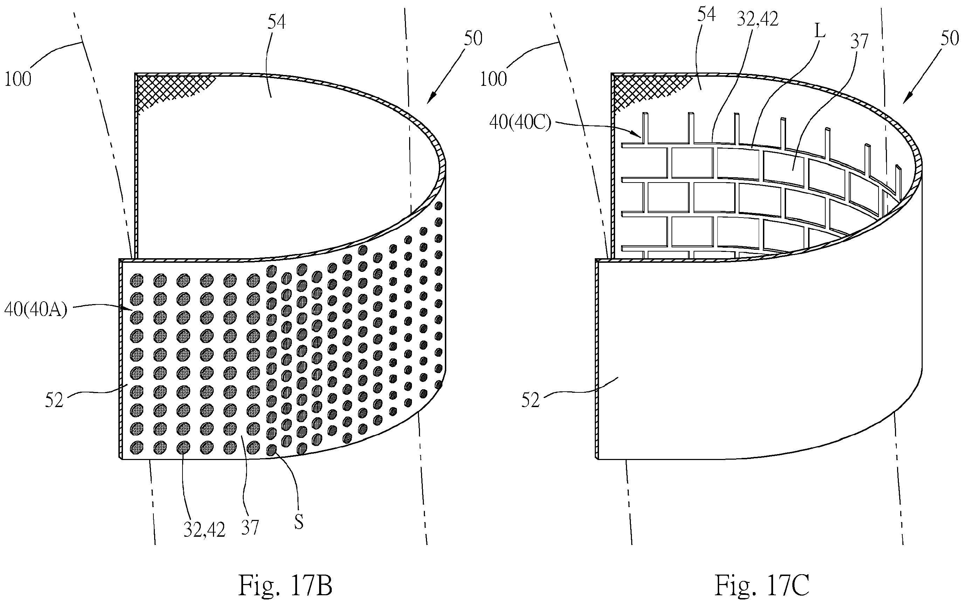

FIGS. 17A to 17C illustrate application examples of the supporting mechanism 40 of the invention fabricated on textile 50 (such as fabrics, clothes, trousers, socks, or protective gear such as elbow pads and knee pads), which are not limited thereto. FIGS. 17A and 17B show that the outer surface 52 of the textile 50 is provided with at least one non-line-shaped supporting mechanism, such as the supporting mechanism 40A or the supporting mechanism 40B or is provided with the supporting mechanisms 40A and 40B. FIGS. 17A and 17C show that the inner surface 54 of the textile 50 has at least one line-shaped supporting mechanism 40C. When the human body wears a fitness garment made of the textile 50, the supporting mechanism 40 (40A to 40C) on the fitness garment elastically wraps the muscles, and a stretch recovery of the supporting mechanism 40 enables the textile 50 to provide elastic pressure and elastic restraint effects to a wearer to support muscles, joints and bones of the human body. For example, when an athlete performs stretching exercises such as squat, the supporting mechanism 40 on fitness trousers is capable of helping the muscles of the thighs to recover, so that performing fitness exercise is more labor-saving, and the supporting mechanism 40 also avoids injury to the motor organs. The supporting mechanism 40A or/and 40B on the outer surface 52 can be designed into any shape and pattern according to characteristics or requirements of garments, such as the pattern in FIG. 10; while the supporting mechanism 40C on the inner surface 54 is in contact with the skin, since the supporting mechanism 40C is made of a polymerized material and has anti-slip function, so the supporting mechanism 40C is capable of ensuring that garments, trousers or protective gear will not slide on the human body, thereby ensuring that the supporting mechanism 40 is kept at a position where the human muscle tissues and other motor organs are supported or protected.

Application examples of the textile 50 with the supporting mechanism 40 of the invention are further illustrated below. The textile 50 can be made into various wearables for the human body to wear, such as clothes, trousers, socks, silk stockings, shoes, bra cups and protective gear. The supporting mechanism 40 on the textile 50 covers the torso, muscles, joints, core muscle groups of the human body to provide support and protection.

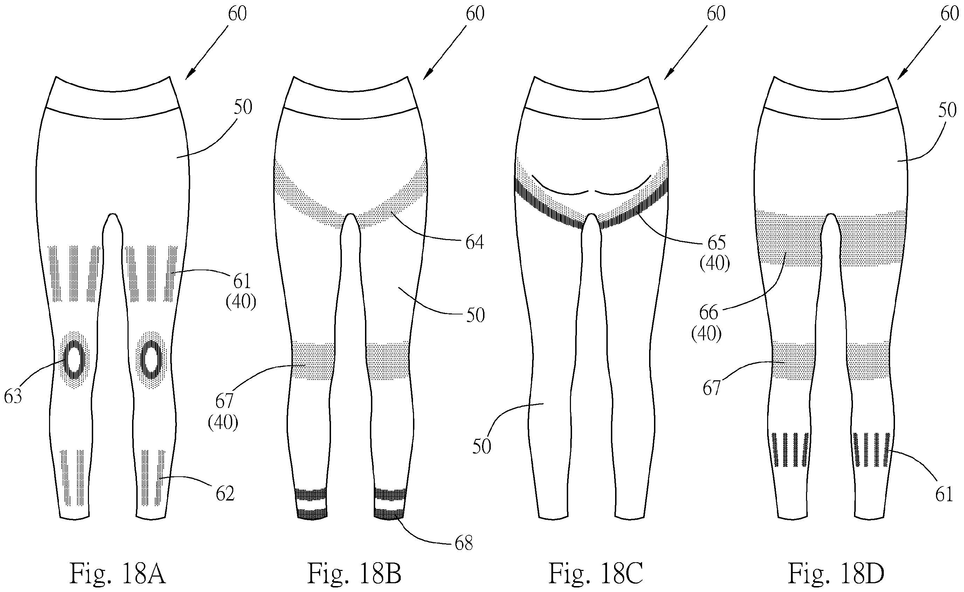

As shown in FIG. 18A to FIG. 18D, the textile 50 of the invention can be made into pressure garments, trousers or fitness garments and trousers. Taking pressure trousers or fitness trousers 60 as an example, the supporting mechanism 40 (the elastic support 42 and the printed layer 32) is disposed on an outer surface and/or an inner surface of the fitness trousers 60, and is made into support strips 61, 62 of the fitness trousers 60 to wrap and support the thighs and the calves, or is made into a ring-shaped wrapping area 63 to wrap and protect the knees. The supporting mechanism 40 can also be made into an oblique support strip 64 on a front of the fitness trousers 60 to protect the hips; or made into an oblique support strip 65 on a back of the fitness trousers 60 to lift the buttocks; or made into wrapping rings 66, 67, 68 along leg parts of the fitness trousers 60 to wrap and protect the thighs, knees, calves and ankles. When a bodybuilder wears the fitness trousers 60 for fitness exercise, such as squatting, the supporting mechanism 40 of the textile 50 elastically wraps or restrains the muscles, knees, ankles, and stabilizes the core muscle groups, preventing the user's muscles and joints from being injured or strained. Elastic recovery of the supporting mechanism 40 enables users to exercise more effortlessly and safely. The supporting mechanism 40 on the inner surface of the fitness trousers 60 not only provides a support function, but also has an anti-slip effect, so that the fitness trousers 60 will not move freely on the human body, and the supporting parts (61-68) formed by the supporting mechanism 40 are kept in their supporting positions.

FIG. 19 shows a schematic diagram of the textile 50 of the invention applied to a pressure garment, a fitness garment or a sportswear 69. The supporting mechanism 40 of the invention can be disposed on an inner surface and/or an outer surface of the sportswear 69, and is disposed on a body part or sleeves of the sportswear 69 to support the torso or arms of a wearer. In FIG. 19, the supporting mechanism 40 disposed on a sleeve 691 is used as an example, and the sportswear 69 is suitable for basketball or baseball players to support and protect the arms.

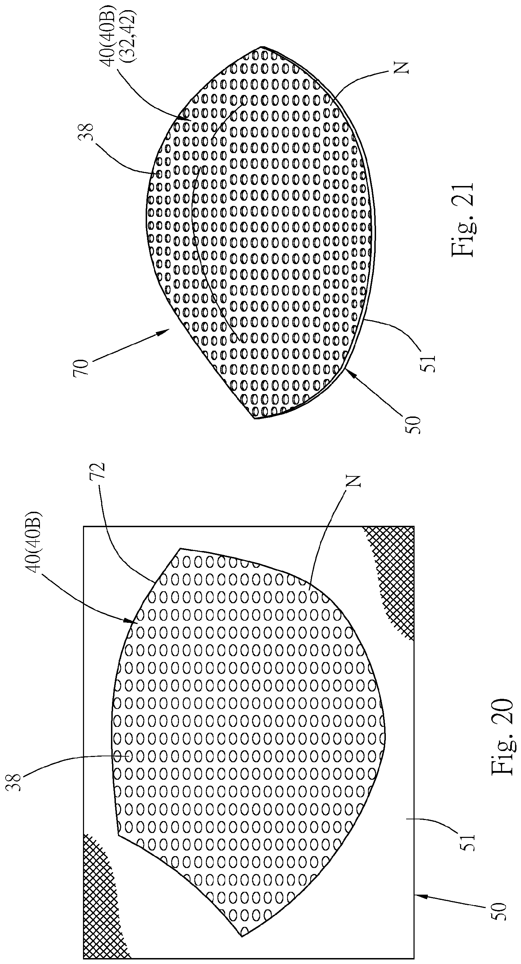

The textile 50 of the invention is suitable for making bra cups. As shown in FIG. 20, according to an outline of a bra cup, a semi-finished product 72 of the bra cup with the supporting mechanism 40 is made on one surface of the textile 50, and then the semi-finished product 72 is cut and hot-pressed and shaped into a bra cup 70 with a mold, as shown in FIG. 21. Production speed of the bra cup 70 is fast, the air-permeable elements (air-permeable holes 38) of the supporting mechanism 40 enable the bra cup 70 to have excellent air permeability, and the supporting mechanism 40 enables the bra cup 70 to keep in shape and provides support for the breast reliably. Inner and outer surfaces of the bra cup 70 can be provided with the supporting mechanism 40, and a lower half of the bra cup 70 can be provided with the supporting mechanism 40 with a higher density or higher mass to support the breast.

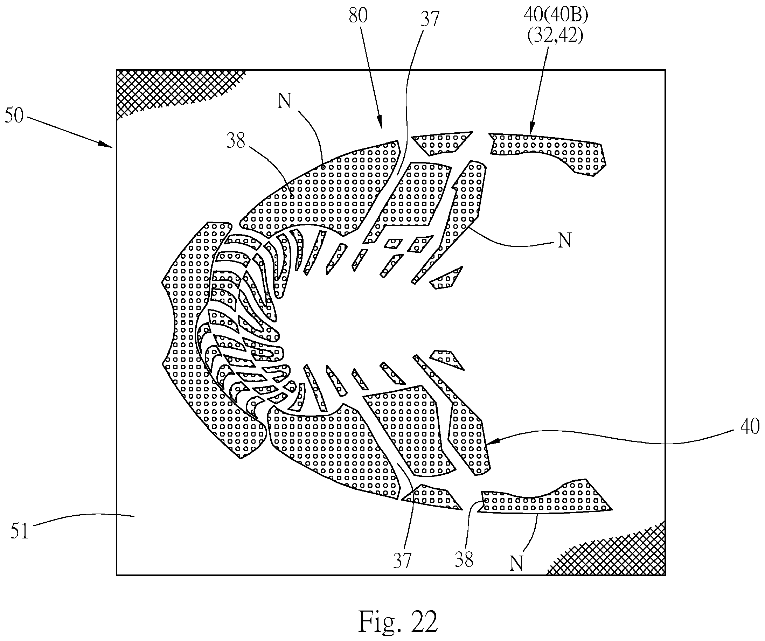

The textile 50 of the invention can be applied to make shoes. As shown in FIG. 22, according to size and style of a vamp 80, the textile 50 is made into the vamp 80 with the supporting mechanism 40, and then the vamp 80 is cut. The vamp 80 is combined with a sole to make a shoe. Production speed of the shoe is fast, the supporting mechanism 40 provides support for the shoe, and each part of the vamp 80 can be made into the supporting mechanism 40 with different densities and different support strengths according to different support degrees. Air can easily circulate in and out of the shoe through the air-permeable elements (air-permeable holes 38). The supporting mechanism 40 of this embodiment is disposed on the vamp 80 by arranging as a purposeful support block, a shape of the support block is approximately symmetrical on upper and lower parts when being viewed based on directions of FIG. 15. Similarly, inner and outer surfaces of the vamp 80 can be provided with the supporting mechanism 40, and the supporting mechanism 40 on the inner surface is in contact with a foot to reduce sliding between the shoe and the foot.

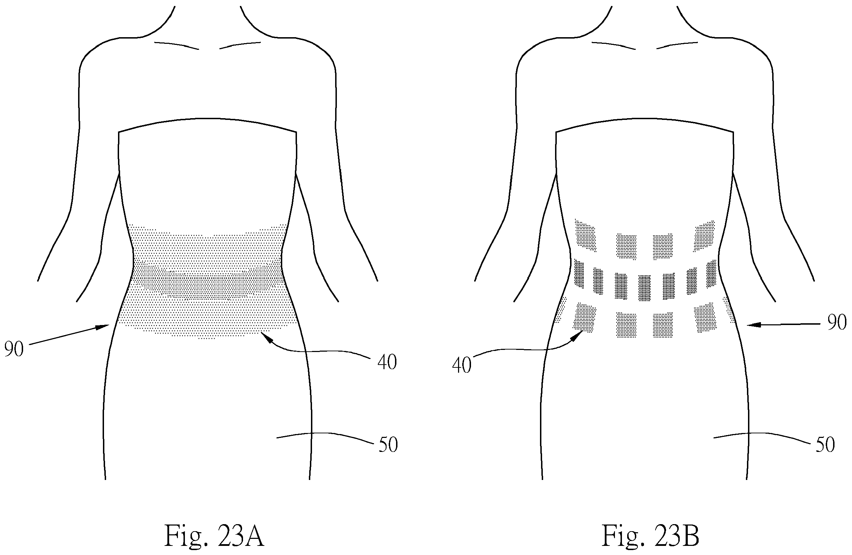

The textile 50 of the invention can also be used to make body-shaping garments. As shown in FIGS. 23A and 23B, the textile 50 is made into a garment 90 with a body-shaping function, and the supporting mechanism 40 is located at a specific position, for example, on a waist of the garment 90. The supporting mechanism 40 is capable of restraining the waist and concentrating the skin and muscles toward the breasts in order to achieve an effect of body shaping.

The textile 50 provided by the invention can be made into various wearables for people to wear, and the supporting mechanism 40 can be customized for the wearables. The supporting mechanism 40 of the textile 50 comprises the elastic support 42 for providing support and the printed layer 32 for forming patterns and colors. The supporting mechanism 40 has a fast manufacturing speed, and pattern, color, size, shape and outline of the supporting mechanism 40 can be manufactured according to design. Thickness and support strength of the supporting mechanism 40 can be adjusted or changed as required.

The elastic support 42 of the supporting mechanism 40 provides support for wearables, and has effects of recovery elasticity and wear resistance. The supporting mechanism 40 can be made into clear patterns and 3D patterns. The air-permeable elements enable the textile 50 to have excellent air permeability.

Various wearables made of the textile 50 of the invention have efficacies of energy saving, carbon reduction, manufacturing cost reduction and pollution reduction.

Taking manufacture of shoes as an example, when manufacturing the vamp 80 according to the invention, the thermoplastic elastomer material layer 36 and the printed layer 32 in the thermoprinting material 10 need to be hot stamped on the textile 50 to form the supporting mechanism 40, and then the vamp 80 can be cut out. The printed layer 32 is directly printed with a color of the vamp 80, and each part of the vamp 80 can be printed with a different color. Shoes made by the invention can replace leather shoes and sports shoes. Support of the supporting mechanism 40 on the vamp 80 is comparable to a wrapping effect of a leather shoe, and its air permeability is close to that of a shoe made of knitted cloth. A color of the vamp 80 is physically combined with the fibers of the textile 50 without dyeing, and there is no problem of environmental pollution caused by dyeing process. That is, compared with the conventional dyeing process, a color of the supporting mechanism 40 is formed by printing, no water is used, no water resources are wasted, no industrial waste water is produced, no additional equipment is required to process industrial waste water, and no pollution is caused. Furthermore, production speed of the invention is fast, and production method thereof is environmentally friendly. Compared with the manufacturing process of leather shoes, the vamp 80 of the invention does not need to cut out several pieces and then join the pieces, and no dyeing is required, process steps and manpower usage are greatly reduced, energy saving and carbon reduction are achieved, and the invention also solves the pollution problem of recycling leather. As for sports shoes, only one piece of fabric is needed for the vamp 80 of the invention. Compared with structure and manufacturing process of the conventional sports shoes, there is no need to sew multiple layers of fabrics or dye the fabrics. The invention also has effects of reducing processes, simplifying manufacturing, without producing pollution, reducing labor usage, energy saving, carbon reduction and reducing manufacturing costs.

When the invention is used to make pressure garments and trousers (elastic garments, trousers), the supporting mechanism 40 of the textile 50 provides an excellent support effect for the muscles, joints, core muscle groups and bones of the human body, and reduces a chance of injury to users during exercise. Fitness garments made of the invention have excellent air permeability and are easy to put on and take off. Similarly, when the invention is applied to fitness garments, it also has an effect of energy saving and carbon reduction, that is, processes such as cutting of plastic materials and sewing are not required, and no dyeing is required. The textile 50 of the invention does not require elastic fibers, or only uses a small amount of elastic fibers (e.g., 2%), which can reduce costs.

When the invention is applied to a bra cup, the supporting mechanism 40 only needs to be fabricated on a piece of fabric, and then shape it into a shape of the bra cup, which reduces various manufacturing processes and manpower, and greatly increases a production speed of the bra cup. Compared with the conventional bra cups, the invention can greatly reduce a unit weight of the fabric under the same supporting force. Conventional bra cups need to be shaped at extremely high temperature (185-195 degrees), which will cause yarns to crack. If the conventional bra cups are made of dyed and finished fabrics, dyeing of the yarns will have a great impact. In the invention, the bra cup 70 is hot-pressed and shaped at a low temperature (125°C), thermal sublimation is reduced, a color of the bra cup 70 is not distorted, and cracking of yarns is slight.

The invention can be applied to socks, silk stockings, pantyhose and other wearables for feet and legs to make an elastic stocking with the supporting mechanism 40, and the supporting mechanisms 40 are specifically arranged on the elastic stocking to form elastic pressure differences at different positions of the stocking, thereby guiding blood flow, so that the elastic stocking can be used to prevent and treat varicose veins.

In the invention, the supporting mechanism 40 is combined after the fabric 51 is made, and the supporting mechanism 40 is combined on the fabric 51 at a low temperature, so that the fibers of the fabric 51 will not crack.

Shape, size, and support strength of the supporting mechanism 40 of the invention can be changed at will, so that wearables of the human body can produce different structural supports. When the supporting mechanism 40 has components such as graphene or collagen, graphene can generate far infrared rays, increase blood oxygen level and promote blood circulation; and collagen has an excellent moisturizing function, which can promote moisturizing and whitening effects of human skin. The textile 50 of the invention can also be made into wearables for animals to assist various animals such as dogs, cats, horses, cows in supporting their motor organs, or assist their limb movement or rehabilitation.

The textile provided by the invention solves various deficiencies in the prior arts. The embodiments disclosed in the invention are only intended to illustrate the technical means of the invention rather than limiting them, and all equivalent modifications of the invention should be regarded as the protection scope of the invention.

## Claims

1. A textile for supporting human motor organs, comprising:
a fabric made by weaving;
at least one supporting mechanism combined onto at least one surface of the fabric, the supporting mechanism having a printed layer and a layered elastic support;
the printed layer being formed by resin printing;
the elastic support being made of thermoplastic elastomer, its shape being consistent with the printed layer, and being combined with the printed layer; the printed layer and the elastic support being combined on the fabric; and
a plurality of air-permeable elements densely distributed within a disposing range of the supporting mechanism.

2. The textile as claimed in claim 1, wherein the fabric has two surfaces; and the at least two supporting mechanisms are respectively combined with the two surfaces of the fabric.

3. The textile as claimed in claim 1 or 2, wherein the supporting mechanism is formed by non-line-shaped supports or line-shaped supports, and the support has the printed layer and the elastic support.

4. The textile as claimed in claim 1 or 2, wherein the supporting mechanism is formed by a plurality of adjacent monolithic supports, each of the monolithic supports has the printed layer and the elastic support, and an air-permeable gap is disposed between the two adjacent monolithic supports.

5. The textile as claimed in claim 1 or 2, wherein the supporting mechanism is at least one area-shaped support, the area-shaped support has the printed layer and the elastic support; and a plurality of air-permeable elements are located within a range of the supporting mechanism.

6. The textile as claimed in claim 5, wherein the supporting mechanism has a plurality of area-shaped supports; and an air-permeable gap is disposed between the two adjacent area-shaped supports.

7. The textile as claimed in claim 1 or 2, wherein the supporting mechanism is formed by at least one line-shaped support, the line-shaped support has the printed layer and the elastic support; and a plurality of air-permeable gaps are located within a range of the line-shaped support.

8. The textile as claimed in claim 7, wherein the line-shaped support is formed by lines in at least two directions, and the air-permeable gaps are located between the lines.

9. The textile as claimed in claim 8, wherein the lines in at least two directions are connected to one another to form a plurality of grids.

10. The textile as claimed in claim 8, wherein an area of the air-permeable gaps is larger than an area of the lines.

11. The textile as claimed in claim 1 or 2, wherein the textile has an outer surface and an inner surface, the inner surface contacts a user of the textile; and the at least one supporting mechanism is provided on either the inner surface or the outer surface.

12. The textile as claimed in claim 1 or 2, wherein the textile has an outer surface and an inner surface, the inner surface contacts a user of the textile; and comprises at least two said supporting mechanisms, wherein at least one of the supporting mechanisms is provided on the outer surface; and at least one of the supporting mechanisms is provided on the inner surface, which is a line-shaped support having the printed layer and the layered elastic support, a plurality of air-permeable gaps are disposed in the line-shaped support.

13. The textile as claimed in claim 1 or 2, wherein the textile is a fabric or a wearable made of a fabric.

14. The textile as claimed in claim 1 or 2, wherein the thermoplastic elastomer is polyurethane thermoplastic elastomer (TPU), polyamide thermoplastic elastomer (TPAE), polyester thermoplastic elastomer (TPEE) or polyolefin thermoplastic elastomer (TPO).

15. The textile as claimed in claim 1 or 2, wherein the printed layer is a thermoplastic high molecular elastic polymer and has colors or is colorless.

16. The textile as claimed in claim 1 or 2, wherein the elastic support is added with graphene or collagen.

17. A supportive thermoprinting material comprising:
a substrate, one surface of the substrate being a release surface;
a printed layer disposed on the release surface of the substrate, the printed layer being formed by printing; a plurality of air-permeable elements being formed within a printing range of the printed layer; and
a thermoplastic elastomer material layer capable of generating adhesion when being heated, the thermoplastic elastomer material layer being disposed on the printed layer and without covering the air-permeable elements.

18. The thermoprinting material as claimed in claim 17, wherein the thermoplastic elastomer material layer is combined with the printed layer in a powder or granular form.

19. The thermoprinting material as claimed in claim 18, wherein the thermoplastic elastomer material layer is combined with the printed layer before the printed layer becomes dry.

20. The thermoprinting material as claimed in claim 17 or 18, wherein the printed layer is a plurality of monolithic printed bodies, or area-shaped printed bodies, or line-shaped printed bodies, and the air-permeable elements are air-permeable holes or air-permeable gaps.

21. The thermoprinting material as claimed in claim 17 or 19, further comprising a bonding promotion layer disposed on the release surface of the substrate; and the printed layer being printed on the bonding promotion layer.

22. The thermoprinting material as claimed in claim 17 or 18, wherein the printed layer has polyurethane and a pigment, and the pigment is colored or colorless.

23. The thermoprinting material as claimed in claim 17 or 18, wherein the printed layer comprises a thermoplastic high molecular elastic polymer; the thermoplastic elastomer material layer is polyurethane thermoplastic elastomer (TPU), polyamide thermoplastic elastomer (TPAE), polyester thermoplastic elastomer (TPEE) or polyolefin thermoplastic elastomer (TPO).
